# EUROPEAN PATENT SPECIFICATION

(11) **EP 4 505 343 B1**
(45) Date of publication and mention of the grant of the patent: **26.08.2026**
(21) Application number: 23713907.6
(22) Date of filing: 24.03.2023
(51) Int. Cl.: G06F 21/62, G16H 10/60, G16H 20/40

(54) **METHOD FOR ANONYMIZING AN AUDIO DATA STREAM**
VERFAHREN ZUR ANONYMISIERUNG EINES AUDIODATENSTROMS
PROCÉDÉ D'ANONYMISATION D'UN FLUX DE DONNÉES AUDIO

(30) Priority: 01.04.2022 NL 2031478
(43) Date of publication of application: 12.02.2025
(73) Proprietor: DEO N.V., 3583 Beringen (BE)
(72) Inventor: DILLE, Jeroen Willem Rita, 3583 BERINGEN (BE); FESTJENS, Niels, 3583 BERINGEN (BE)
(74) Representative: Arnold & Siedsma
(86) International application number: PCT/EP2023/057665
(87) International publication number: WO 2023/186742

(56) References cited:
- US-A1- 2017 249 432
- US-A1- 2021 012 032
- COHEN-HADRIA ALICE ET AL: "Voice Anonymization in Urban Sound Recordings", 2019 IEEE 29TH INTERNATIONAL WORKSHOP ON MACHINE LEARNING FOR SIGNAL PROCESSING (MLSP), IEEE, 13 October 2019 (2019-10-13), pages 1 - 6, XP033645862, DOI: 10.1109/MLSP.2019.8918913

## Description

The field of the invention relates to a method for anonymizing an audio data stream. The invention further relates to a computer program product for executing said method and a system for anonymizing an audio data stream in a hospital environment.

Hospital environments or, more generically medical environments, are subject to optimization processes, for example optimizing the use of equipment during an operation. Machine learning solutions or optimization algorithms may be modelled to recognize patterns, interpret data and cluster or label raw input data. However, such solutions require a specific set of training data which is not readily available. For privacy and/or security reasons, the inner workings of hospital environments are not publicly disclosed. In particular patient details are securely protected and such patient details are not usually required for process optimization.

In order to obtain the training data, it is thus suggested to use audio data. Audio recording means are arranged to gather insights into the on-going processes for example in an operating room. However, the obtained audio data stream must be anonymized before it is for example transferred over the internet and further utilized for e.g. for optimizing processes in the hospital environment. US patent application 2021/0012031 A1 discloses a method for anonymizing data in a surgical robotic system. US patent application 2017/024932 A1 discloses a multi-channel recorder/encoder for collecting, integrating, synchronizing and recording medical or surgical data.

The object of embodiments of the present invention is to provide an improved method for anonymizing audio data, for instance in a more secure and/or efficient way. The invention is defined by the appended claims.

According to a first aspect of the present invention there is provided a method for anonymizing an audio data stream in a hospital environment, the method comprising:
- obtaining audio data using an audio recording means in the hospital environment, wherein the audio data comprises of one or more audio signals;
- determining at least one sound of interest, SOI, within the one or more audio signals comprised in the audio data;
- detecting if a SOI comprises speech identification features which allow identification of a person in the hospital environment;
- anonymizing at least the audio signal comprising the SOI comprising the detected speech identification features;
- outputting the anonymized audio data.

It is thus suggested that audio data can be used as input, for instance as training data, in an optimization process. Audio recording means may thus be arranged to gather insights in the on-going processes in for example an operating room. By anonymizing the obtained audio data stream, it is possible to be used for optimizing processes in the hospital environment. Obtaining audio data using an audio recording means in the hospital environment allows obtaining real life sounds of medical and non-medical personal and peripheral sounds, for example a heart monitor sound or hammer sound. The audio data may be used for generating information about the operating room process in of itself or in combination with video data. It may be used to identify time stamps, presence of personal or equipment and provide information on the use of the equipment.

The advantage of obtaining audio data, determining a Sound of Interest, abbreviated as SOI, and detecting if a SOI comprises speech identification features is based on the insight that for audio signals personal identification features are limited to the speech of a respective person, i.e. sustained vowels sounds or timbre of the voice. Identification features are, in the current context, preferably defined as a physical identifying feature, such as a voice of a person such as a surgeon, surgical staff or patient. In other words, identification features may represent physical characteristics which allow the identification of a human in the hospital environment. This is distinguishable from other physical components present in the hospital environment, such as the previously mentioned heart monitor equipment. Limiting the anonymization to the SOI comprising the speech identification features rather than perform further processing on the entirety of the audio data allows the method to efficiently output anonymized audio data. Moreover, anonymizing at least the audio signal comprising the SOI comprising the detected speech identification features allows to retain valuable peripheral sounds, such as the equipment sounds mentioned above. This also allows to interpret the audio data further without disclosing privacy information of patients or medical personnel.

As mentioned, the method preferably comprises the step of obtaining audio data using an audio recording means in the hospital environment. Any suitable recording means can be used. It may however also be possible that the method comprises the general step of obtaining audio data recorded using a recording means in the hospital environment. Audio data, for instance a previously recorded audio file or obtained from a video file, may then be analysed as described by determining any sound of interest etc.

Preferably, the method further comprises isolating the audio signal comprising the detected speech identification features from the obtained audio data. Anonymization can be performed in a plurality of ways, for example by muting a time portion of the complete audio data where speech is recognized. This is relatively simple and provides a robust way of anonymizing privacy information of people present. However, by anonymizing a time portion of the complete audio data a portion of peripheral sounds are also anonymized and therefore unavailable as training data or for other further processing steps. By isolating the audio signal comprising the detected speech identification features from the obtained audio data one or more further audio signals are retained and their correspondingly comprised Sounds of Interests may be used in future and further processing steps, for example as training data.

Preferably, the anonymizing comprises subtracting the isolated audio signal from the obtained audio data.

Preferably, the method further comprises training a speech recognition system using the isolated audio signal. More preferably, the step of detecting if a SOI comprises speech identification features is performed using the trained speech recognition system. The trained speech recognition system may be used in turn to improve and/or increase processing speed of detecting if a SOI comprises speech identification features. Moreover, when the trained speech recognition is used in a known environment, the trained speech recognition system will more easily recognize known voices of, for example, surgeons or medical personal without the threat of multiplying sensitive privacy data. Also, the recognition of SOI comprising speech identification features is more robust and reliable.

Preferably, the anonymizing comprises muting a portion of the audio data comprising the SOI comprising the detected speech identification features. Anonymizing in the current context may also be referred to as muting or attenuating the audio signal in which the respective audio signal is silenced or attenuated below a predetermined threshold.

Preferably, the method further comprises detecting if a SOI comprises tool audio features. More preferably, the method further comprises classifying the audio signal comprising the tool audio features. More preferably, the method comprises outputting the classified audio signal. As mentioned here above, tool audio features, such as a heart rhythm originating from a heart monitor, provides valuable information in optimizing care and processes in a hospital environment. Classifying the audio signal allows the method to identify the tools used in the hospital environment. Outputting the classified audio signal comprising the tool audio features allows the outputted audio signal to be used as a training data set which in turn can be used to optimize the method further. More preferably, the classifying comprises annotating the audio signal with any one of the following parameters: use of tool, duration of use, frequency, force of use, variation of force of use.

The method may thus include a step of determining at least one tool parameter, such as use of tool, duration of use, frequency, force of use, variation of force of use, on the basis of the SOI comprising tool audio features. The method may then comprise outputting the determined tool parameter. Outputting the tool parameter may take place together with the anonymized audio data, for instance by annotation as mentioned above, or as alternative to the step of outputting the anonymized audio data.

Preferably, the method further comprises detecting if a SOI comprises cardiac audio features. More preferably, the method comprises measuring a cardiac rhythm based on the detected audio signal comprising the cardiac audio features. More preferably, the method further comprises outputting the audio signal comprising the detected cardiac audio features.

Also here, the method may include a step of determining a cardiac parameter, such a rhythm and/or other relevant cardiac parameter and outputting the determined cardiac parameter. Outputting may take place together with or as an alternative to the outputting of anonymized audio data.

In general, the method may include determining at least one process parameter, such as the tool or cardiac parameter as mentioned above, from the audio data, or specifically a SOI thereof. The method then preferably comprises the step of outputting the determined process parameter in addition to or as an alternative to the outputted anonymized audio data.

It may for instance be the case that the audio data at the same time comprises both a SOI comprising speech data, or other audio data that needs to be anonymized, and relevant process audio features, for instance cardiac audio features. If the audio data would be muted, also the relevant process audio parameter would be lost. By also outputting any determined process parameter, this parameter can also be taken into account as training data, for instance.

According to a second aspect of the present invention a computer program product comprising a computer-executable program of instructions for performing, when executed on a computing device, the steps of the method as described here above.

According to a third aspect of the present invention a system for at least partially anonymizing an audio data stream in a hospital environment, the system comprising an audio recording means for obtaining audio data, wherein the audio data comprises one or more audio signals, an audio processor configured to perform, the steps of the method as described here above.

It will be understood by the skilled person that the features and advantages disclosed hereinabove with respect to the method may also apply, *mutatis mutandis,* to the computer program and the system.

According to yet another aspect of the present invention, there is provided a method for downloading to a digital storage medium a computer-executable program of instructions to perform, when executed on a computing device, the steps of the method described above.

The accompanying drawings are used to illustrate presently preferred non-limiting exemplary embodiments of devices of the present invention. The above and other advantages of the features and objects of the present invention will become more apparent and the present invention will be better understood from the following detailed description when read in conjunction with the accompanying drawings, in which:
Figure 1 schematically illustrates a method for anonymizing an audio data stream in a hospital environment according to a preferred embodiment;
Figure 2 schematically illustrates a preferred embodiment of the method shown in Figure 1;
Figure 3 schematically illustrates a preferred embodiment of a method, including, in this example, anonymizing a portion of the audio data;
Figure 4 schematically illustrates a preferred embodiment of a method of figure 1 further developed to output classified audio signals.

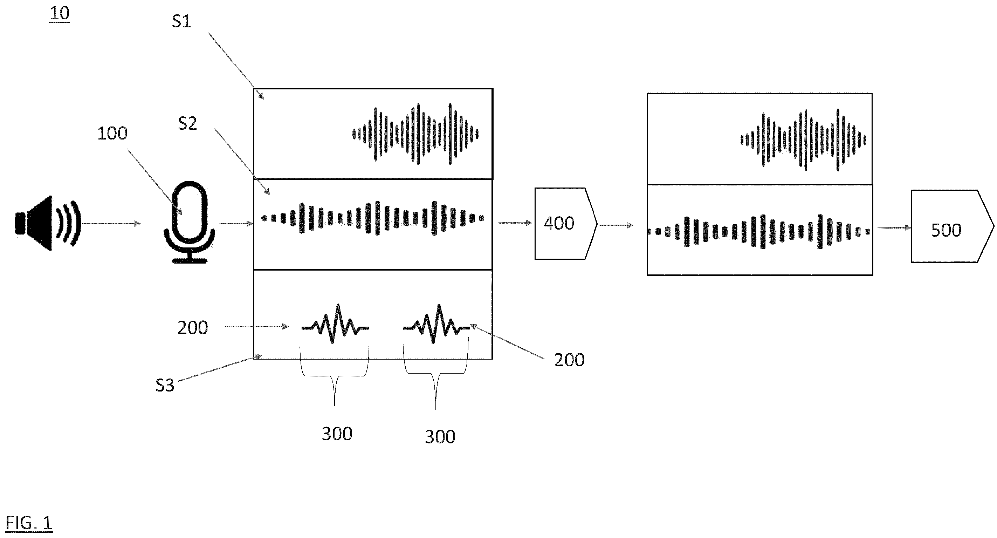

Figure 1 shows a flowchart of a method 10 for anonymizing an audio data stream in a hospital environment. The illustrated method may also at least partially anonymize the audio data stream as will be elaborated here below.

The method 10 comprises obtaining 100 audio data using an audio recording means in the hospital environment, for example an operating room. The audio data originates from a sound source in the hospital environment, for example a surgeon speaking or a surgical drill being used during an operation. For obtaining 100 the audio data, any suitable audio recording means may be used. However, it is preferred that a digital audio recording means is used, for example a digital sound recorder. It is not essential that an audio recording means is used which is exclusively dedicated to audio recording, other suitable devices such as a mobile phone or a video camera may also be used to obtain the audio data. The audio data may also be obtained in combination with video data. It may however also be possible that the method 10 comprises the general step of obtaining 100 audio data recorded using a recording means in the hospital environment. Audio data, for instance a previously recorded audio file or obtained from a video file, may then be analysed as described by determining any sound of interest as will be elaborated here below. In the current context the expression "obtaining audio data" and "recording" may be interchangeably used.

The obtained audio data comprises one or more audio signals S1, S2, S3. In figure 1 the audio signals are illustrated as separate audio waves for illustration purposes. In practice however, the audio signals will overlap in a sample of the audio data. The obtained audio data provides valuable insights regarding the inner workings of the hospital environment. One of the audio signals may for example relate to the audible cues of a heart monitor. Another audio signal may relate to a surgeon requesting tools from a nurse in the operating room and yet another audio signal may simply be a radio providing background music or generic radio chit chat in the operating room. For privacy and/or security reasons, the inner workings of hospital environments are not publicly disclosed. In particular patient details are securely protected and such patient details are not usually required for process optimization. For this reason the audio data must be anonymized.

Within the one or more audio signals S1, S2, S3 comprised in the obtained audio data, the method 10 determines 200 at least one sound of interest. A Sound of Interest, abbreviated as SOI, may be a heart monitor providing audible cues to the medical personnel in the room or may be a person talking, to provide a few examples.

Next the method 10 detects 300 if a SOI comprises speech identification features which allow identification of a person in the hospital environment. Such a person may be a surgeon, nurse or patient. Identification features are, in the current context, defined as a physical identifying feature, such as a voice of a person such as a surgeon or patient. In other words, identification features represent physical characteristics which allow the identification of a human in the hospital environment. In figure 1 audio signal S3 comprises a SOI which has been detected 300 as comprising speech identification features. This is distinguishable from other physical components present in the hospital environment, such as the previously mentioned heart monitor audible cues, which is illustrated in audio signal S1 as an example. Moreover, audio signal S2 may represent a constant background noise present in the hospital environment, for example a fan of an air-conditioning unit providing fresh air in the operating room or a radio providing background entertainment for the patients or staff in the hospital environment.

Further, at least the audio signal S3 comprising the SOI comprising the detected 300 speech identification features is anonymized 400. Limiting the anonymization 400 to the SOI comprising the detected 300 speech identification features rather than perform further processing on the entirety of the audio data allows the method to efficiently output anonymized audio data. Moreover, anonymizing 400 at least the audio signal comprising the SOI comprising the detected speech identification features allows to retain valuable peripheral sounds, such as the equipment sounds mentioned above. This also allows to interpret the audio data further without disclosing privacy information of patients or medical personnel. Finally, the anonymized audio data is output 500.

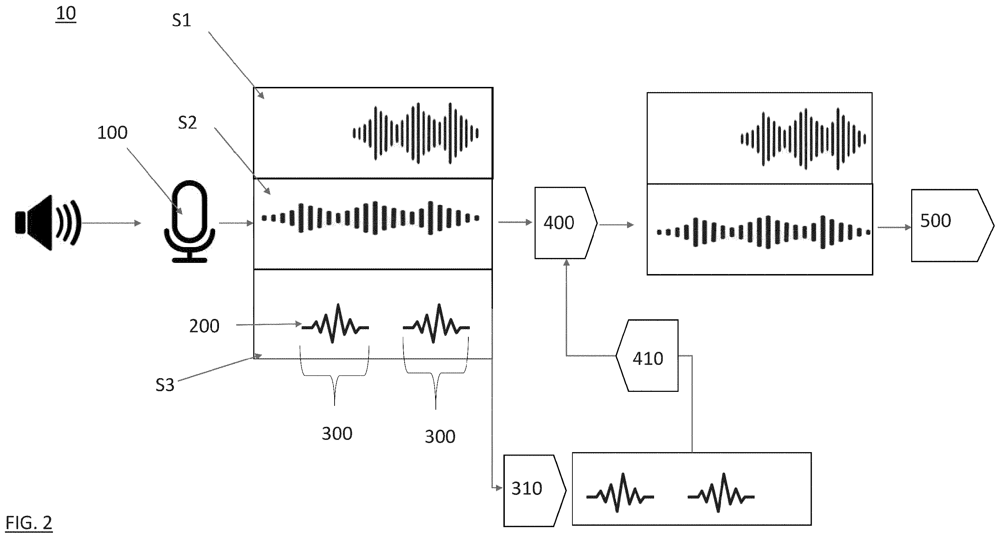

Figure 2 illustrates a flowchart of the method of figure 1 being further developed to isolate 310 the audio signal S3 comprising the detected speech identification features from the obtained audio data, according to a preferred embodiment. Isolating 310 the audio signal S3 comprising the detected 300 speech identification features from the obtained audio data is advantageous for a plurality of reasons.

Firstly, the isolated 310 audio signal S3 can be used to train a speech recognition system (not shown). In this way, the step of detecting 300 if a SOI comprises speech identification features may be performed using the trained speech recognition system to improve and/or increase processing speed of detecting 300 if a SOI comprises speech identification features. Moreover, when the trained speech recognition is used in a known environment, i.e. where previously obtained audio data from the same hospital environment is used to train the speech recognition system, the trained speech recognition system will more easily recognize known voices of, for example, surgeons or medical personal forming a part of the regular staff of the hospital environment. This is particularly advantageous as it reduces the threat of multiplying sensitive privacy data. Also, the recognition of a SOI comprising speech identification features of known people is more robust and reliable in comparison to hospital environments where voices of the personnel is not known, for example when the method is performed for a first time in a new hospital environment. Such a speech recognition system is additionally beneficial in the challenge of distinguishing a background radio from speech. Operating rooms often have background music or a radio, which can significantly increase false positives if all music and radio chatter is removed. Speech recognition can be used to distinguish between a radio voice and the voice of a person present in the room. As mentioned here above, the speech recognition system is trained using the audio signal comprising voice of the regular staff of the hospital environment. Additionally, the speech recognition is preferably trained to only identifying the voice as a known voice. Once speech recognition system is capable of identifying voices as known voices, any voice which is heard multiple times throughout the recording and which is not recognized, can be assumed to be a radio voice.

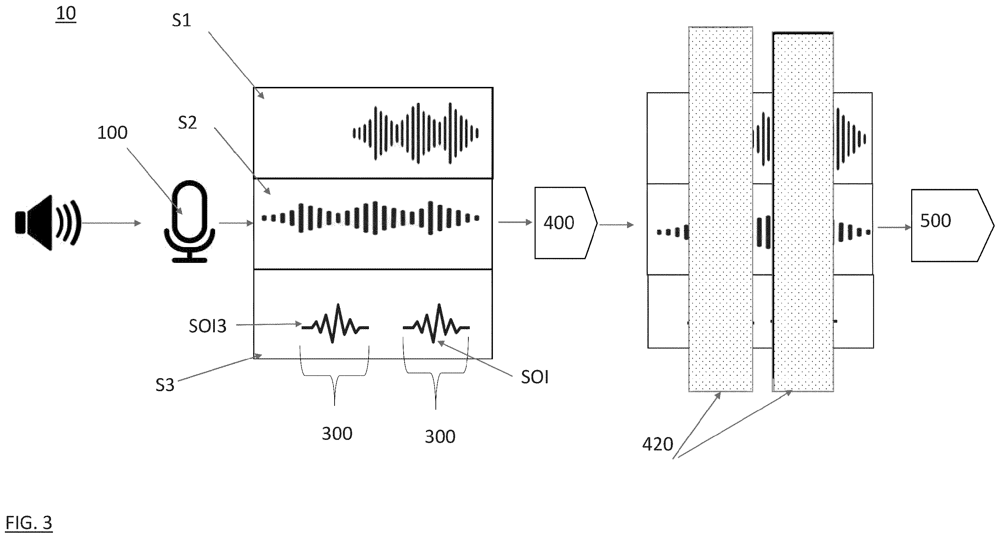

Secondly, anonymization 400 can be performed in a plurality of ways, for example by muting a time portion of the complete audio data where speech is recognized, as will be illustrated in relation to figure 3. This is relatively simple and provides a robust way of anonymizing privacy information of people present. However, by anonymizing a time portion of the complete audio data a portion of peripheral sounds are also anonymized and therefore unavailable as training data. By isolating 310 the audio signal comprising the detected 300 speech identification features from the obtained 100 audio data one or more further audio signals are retained and their correspondingly comprised SOIs may be used in future and further processing steps, for example as the previously mentioned training data. In order to reduce the noise from the audio signal S3 comprising the speech recognition features it is preferred that the anonymizing 400 comprises subtracting 410 the isolated audio signal S3 from the obtained 100 audio data. Subtracting 310 one audio signal S3 from the obtained 100 audio data can be performed in a plurality of ways, for example by inverting the isolated audio signal S3 and overlaying the inverted isolated audio signal on the obtained 100 audio data, effectively cancelling the isolated audio signal S3 from the obtained audio data and anonymizing 400 the audio data as illustrated in figure 2.

Figure 3 illustrates a flowchart of the method of figure 1 being further developed to mute 420 a portion of the audio data comprising the detected speech identification features. A portion of the audio data may be an interval of an audio signal between two time-stamps of said audio signal. For example, when it is detected 300 that a SOI comprises speech identification features between 5 seconds after initiating recording and 10 seconds, the interval 5s-10s may be muted or attenuated. It will be clear that a plurality of intervals may be muted, for example two intervals as illustrated in figure 3. It is noted that, as illustrated in figure 3, the muting may be performed on all of the audio signals at the interval where speech recognition features are detected. Muting or attenuating the audio signal is defined as silencing or attenuating the audio signal below a predetermined threshold. By muting 420 the portion of audio data comprising the detected 300 SOI comprising the detected speech identification features, a robust way of obtained anonymized audio data is obtained. Although figure 3 illustrates that all three audio signals are muted at the portion comprising the detected speech identification features, it is noted that also only the audio signal S3 comprising the detected speech identification features may be anonymized by only muting said portion of the audio signal S3 comprising the speech identification features.

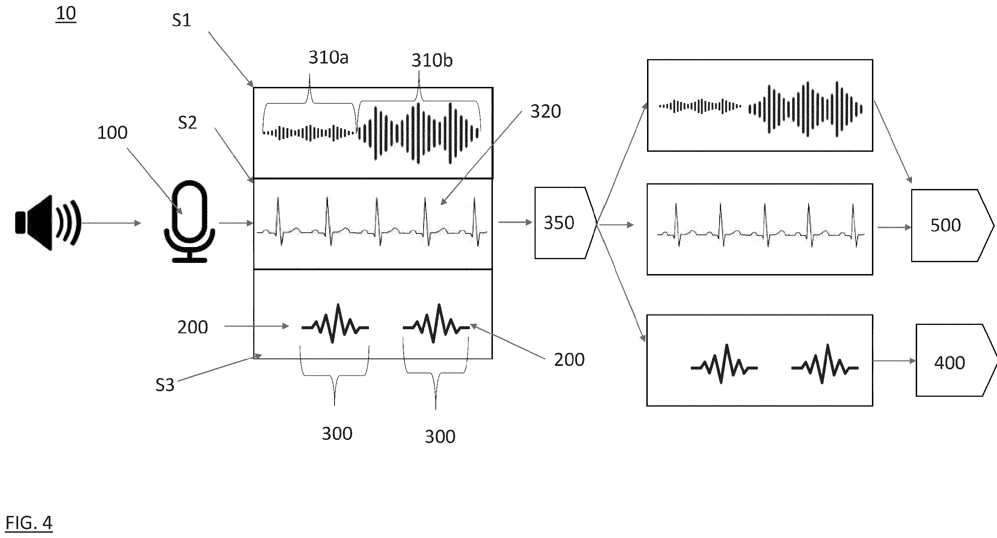

Figure 4 illustrates a flowchart of the method of figure 1 being further developed to detect if a SOI comprises tool audio features 310a, 310b, 320. In figure 4, audio signal S1 is illustrated as an audio signal comprising tool audio features originating from for example a handheld mallet during a knee surgery in an operating room. Audio signal S2 illustrates a cardiac pulse which is typically monitored using a heart monitor in the operating room. Audio signal S3 illustrates a conversation occurring in the operating room, for example a surgeon instructing a nurse, which are speech identification features 300. More preferably, the method further comprises classifying 350 the audio signal comprising the tool audio features. As mentioned here above, tool audio features, such as a heart rhythm 320 originating from a heart monitor, provides valuable information in optimizing care and processes in a hospital environment. The heart rhythm 320 can be used to identify the period during which surgery is happening, as well as identify correlations between heart rate changes and surgical activities. Moreover, various tools make distinct sounds, such as mallet in audio signal S1 or chisels, power tools such as a saw or drill. Classification 350 can be used to identify the moment of use of such tool, the duration, frequency and other device specific properties such as for instance force and force variation when using a mallet. Preferably, the classifying comprises annotating the audio signal with any one of the following parameters: use of tool, duration of use, frequency, force of use, variation of force of use. Having access to these properties can reveal correlations between the action of using the tool and clinical results, as well as impact on staff ergonomics to prevent stress-induced injuries. For instance, impacts of the mallet with high force might be correlated with changes in patient recovery time, as well as physical fatigue of the mallet operator. At the same time, a variation in the frequency at which the mallet is tapped on a chisel, can be used as indicator of operator fatigueness. Although the above method has been explained using a mallet and as mentioned above, other tools create distinct sounds. For example, the action of cauterizing also has a distinct sound. Cauterizing is heard first at the start of the incision, and thus can be used to identify the start of the surgery and specifically the moment of incision. It can also be used to record the amount of cauterization, which is useful for other research such as investigating correlation between cauterization and recovery or long-term effects. Moreover, when surgery is performed using an imaging method such as CT or X-Ray, the moment of recording the image is typically combined with an auditive cue to warn personnel in the operating room. The obtained audio cue can be used to identify the moments and number of images taken, which in turn can be used to estimate radiation exposure for patient and staff, without needing to download information from the scanner or requiring extra sensors. The method may then comprise outputting the determined tool parameter. Outputting the tool parameter may take place together with the anonymized audio data, for instance by annotation as mentioned above, or as alternative to the step of outputting the anonymized audio data. As is illustrated in figure 3, it may for instance be the case that the audio data at the same time comprises both a SOI comprising speech data, e.g. audio signal S3 or other audio data that needs to be anonymized, and relevant process audio features, for instance cardiac audio features such as in audio signals S1 and S2. If the audio data would be muted, also the relevant process audio parameter would be lost. By also outputting any determined process parameter, as is illustrated in figure 4, this parameter can also be taken into account as training data, for instance.

A person of skill in the art would readily recognize that steps of various above-described methods can be performed by programmed computing devices. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The program storage devices may be resident program storage devices or may be removable program storage devices, such as smart cards. The embodiments are also intended to cover computing devices programmed to perform said steps of the above-described methods.

The description and drawings merely illustrate the principles of the present invention. It will thus be appreciated that those skilled in the art will be able to devise various arrangements that, although not explicitly described or shown herein, embody the principles of the present invention and are included within its scope. Furthermore, all examples recited herein are principally intended expressly to be only for pedagogical purposes to aid the reader in understanding the principles of the present invention and the concepts contributed by the inventor(s) to furthering the art, and are to be construed as being without limitation to such specifically recited examples and conditions. Moreover, all statements herein reciting principles, aspects, and embodiments of the present invention, as well as specific examples thereof, are intended to encompass equivalents thereof. The invention is defined by the appended claims.

The functions of the various elements shown in the figures, including any functional blocks labelled as "processors", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, network processor, application specific integrated circuit (ASIC), field programmable gate array (FPGA), Graphics Processing Units (GPUs), read only memory (ROM) for storing software, random access memory (RAM), and non volatile storage. Other hardware, conventional and/or custom, may also be included. Similarly, any switches shown in the figures are conceptual only. Their function may be carried out through the operation of program logic, through dedicated logic, through the interaction of program control and dedicated logic, or even manually, the particular technique being selectable by the implementer as more specifically understood from the context.

It should be appreciated by those skilled in the art that any block diagrams herein represent conceptual views of illustrative circuitry embodying the principles of the present invention. Similarly, it will be appreciated that any flowcharts, flow diagrams, state transition diagrams, pseudo code, and the like represent various processes which may be substantially represented in computer readable medium and so executed by a computing device.

It should be noted that the above-mentioned embodiments illustrate rather than limit the present invention and that those skilled in the art will be able to design alternative embodiments without departing from the scope of the appended claims. In the claims, any reference signs placed between parentheses shall not be construed as limiting the claim. The word "comprising" does not exclude the presence of elements or steps not listed in a claim. The word "a" or "an" preceding an element does not exclude the presence of a plurality of such elements. The present invention can be implemented by means of hardware comprising several distinct elements and by means of a suitably programmed computing device. In claims enumerating several means, several of these means can be embodied by one and the same item of hardware. The usage of the words "first", "second", "third", etc. does not indicate any ordering or priority. These words are to be interpreted as names used for convenience.

In the present invention, expressions such as "comprise", "include", "have", "may comprise", "may include", or "may have" indicate existence of corresponding features but do not exclude existence of additional features.

Whilst the principles of the present invention have been set out above in connection with specific embodiments, it is to be understood that this description is merely made by way of example and not as a limitation of the scope of protection which is determined by the appended claims.

## Claims

1. A method (10) for anonymizing an audio data stream in a hospital environment, the method comprising:
- obtaining (100) audio data using an audio recording means in the hospital environment, wherein the audio data comprises one or more audio signals (S1, S2, S3);
- determining (200) at least one sound of interest, SOI, within the one or more audio signals comprised in the audio data;
- detecting (300) if a SOI comprises speech identification features which allow identification of a person in the hospital environment and detecting if a SOI comprises tool audio features;
- classifying the audio signal (S2) comprising the tool audio features;
- anonymizing (400) at least the audio signal (S3) comprising the SOI comprising the detected speech identification features;
- outputting (500) the anonymized audio data and the classified audio signal.

2. The method according to the previous claim, further comprising isolating (310) the audio signal (S3) comprising the detected speech identification features from the obtained audio data.

3. The method according to the previous claim, wherein the anonymizing (400) comprises subtracting (410) the isolated audio signal (S3) from the obtained audio data.

4. The method according to any one of the claims 2-3, further comprising training a speech recognition system using the isolated audio signal (S3).

5. The method according to the previous claim, wherein the step of detecting if a SOI comprises speech identification features is performed using the trained speech recognition system.

6. The method according to any one of the previous claims, wherein the anonymizing (400) comprises muting (420) a portion of the audio data comprising the SOI comprising the detected speech identification features.

7. The method according to claim 1, wherein the classifying comprises annotating the audio signal with any one of the following parameters: use of tool, duration of use, frequency, force of use, variation of force of use.

8. The method according to any one of the previous claims, further comprising detecting if a SOI comprises cardiac audio features.

9. The method according to the previous claim, comprising measuring a cardiac rhythm based on the detected audio signal comprising the cardiac audio features.

10. The method according to any one of the claims 8-9, further comprising outputting the audio signal comprising the detected cardiac audio features.

11. A computer program product comprising a computer-executable program of instructions for performing, when executed on a computing device, the steps of the method of any one of the previous claims.

12. A system for anonymizing an audio data stream in a hospital environment, the system comprising
- an audio recording means (100) for obtaining audio data, wherein the audio data comprises one or more audio signals (S1, S2, S3);
- an audio processor configured to perform, the steps of the method of any one of the claims 1-10.

## Patentansprüche

1. Verfahren (10) zum Anonymisieren eines Audiodatenstroms in einer Krankenhausumgebung, das Verfahren umfassend:
- Erhalten (100) von Audiodaten unter Verwendung eines Audioaufzeichnungsmittels in der Krankenhausumgebung, wobei die Audiodaten ein oder mehrere Audiosignale (S1, S2, S3) umfassen;
- Bestimmen (200) mindestens eines Geräuschs von Interesse, SOI, innerhalb des einen oder der mehreren Audiosignale, die in den Audiodaten umfasst sind;
- Erfassen (300), ob ein SOI Sprachidentifikationsmerkmale umfasst, die eine Identifikation einer Person in der Krankenhausumgebung ermöglichen, und Erfassen, ob ein SOI Werkzeugaudiomerkmale umfasst;
- Klassifizieren des Audiosignals (S2), umfassend die Werkzeugaudiomerkmale;
- Anonymisieren (400) mindestens des Audiosignals (S3), umfassend das SOI, umfassend die erfassten Sprachidentifikationsmerkmale;
- Ausgeben (500) der anonymisierten Audiodaten und des klassifizierten Audiosignals.

2. Verfahren nach dem vorstehenden Anspruch, ferner umfassend ein Isolieren (310) des Audiosignals (S3), umfassend die erfassten Sprachidentifikationsmerkmale, aus den erhaltenen Audiodaten.

3. Verfahren nach dem vorstehenden Anspruch, wobei das Anonymisieren (400) ein Subtrahieren (410) des isolierten Audiosignals (S3) von den erhaltenen Audiodaten umfasst.

4. Verfahren nach einem der Ansprüche 2 bis 3, ferner umfassend ein Trainieren eines Spracherkennungssystems unter Verwendung des isolierten Audiosignals (S3).

5. Verfahren nach dem vorstehenden Anspruch, wobei der Schritt des Erfassens, ob ein SOI Spracherkennungsmerkmale umfasst, unter Verwendung des trainierten Spracherkennungssystems durchgeführt wird.

6. Verfahren nach einem der vorstehenden Ansprüche, wobei das Anonymisieren (400) ein Stummschalten (420) eines Anteils der Audiodaten umfasst, umfassend das SOI, umfassend die erfassten Sprachidentifikationsmerkmale.

7. Verfahren nach Anspruch 1, wobei das Klassifizieren ein Annotieren des Audiosignals mit einem beliebigen der folgenden Parameter umfasst: Verwendung eines Werkzeugs, Dauer der Verwendung, Frequenz, Kraft der Verwendung, Variation der Kraft der Verwendung.

8. Verfahren nach einem der vorstehenden Ansprüche, ferner umfassend das Erfassen, ob ein SOI kardiale Audiomerkmale umfasst.

9. Verfahren nach dem vorstehenden Anspruch, umfassend ein Messen eines Herzrhythmus basierend auf dem erfassten Audiosignal, umfassend die kardialen Audiomerkmale.

10. Verfahren nach einem der Ansprüche 8 bis 9, ferner umfassend das Ausgeben des Audiosignals, umfassend die erfassten kardialen Audiomerkmale.

11. Computerprogrammprodukt, umfassend ein computerausführbares Programm von Anweisungen zum Durchführen, wenn es auf einer Rechenvorrichtung ausgeführt wird, der Schritte des Verfahrens nach einem der vorstehenden Ansprüche.

12. System zum Anonymisieren eines Audiodatenstroms in einer Krankenhausumgebung, das System umfassend
- ein Audioaufzeichnungsmittel (100) zum Erhalten von Audiodaten, wobei die Audiodaten ein oder mehrere Audiosignale (S1, S2, S3) umfassen;
- einen Audioprozessor, der konfiguriert ist, um die Schritte des Verfahrens nach einem der Ansprüche 1 bis 10 durchzuführen.

## Revendications

1. Procédé (10) d'anonymisation d'un flux de données audio dans un environnement hospitalier, le procédé comprenant :
- l'obtention (100) de données audio à l'aide d'un moyen d'enregistrement audio dans l'environnement hospitalier, dans lequel les données audio comprennent un ou plusieurs signaux audio (S1, S2, S3) ;
- la détermination (200) d'au moins un son d'intérêt, SOI, au sein du ou des signaux audio compris dans les données audio ;
- le fait de détecter (300) si un SOI comprend des caractéristiques d'identification vocale qui permet l'identification d'une personne dans l'environnement hospitalier et le fait de détecter si un SOI comprend des caractéristiques audio d'outil ;
- la classification du signal audio (S2) comprenant les caractéristiques audio d'outil ;
- l'anonymisation (400) d'au moins le signal audio (S3) comprenant le SOI comprenant les caractéristiques d'identification vocale détectées ;
- la sortie (500) des données audio anonymisées et du signal audio classifié.

2. Procédé selon la revendication précédente, comprenant en outre l'isolement (310) du signal audio (S3) comprenant les caractéristiques d'identification vocale détectées à partir des données audio obtenues.

3. Procédé selon la revendication précédente, dans lequel l'anonymisation (400) comprend la soustraction (410) du signal audio isolé (S3) des données audio obtenues.

4. Procédé selon l'une quelconque des revendications 2 à 3, comprenant en outre un entraînement d'un système de reconnaissance vocale à l'aide du signal audio isolé (S3).

5. Procédé selon la revendication précédente, dans lequel l'étape consistant à détecter si un SOI comprend des caractéristiques d'identification vocale est effectuée à l'aide du système de reconnaissance vocale entraîné.

6. Procédé selon l'une quelconque des revendications précédentes, dans lequel l'anonymisation (400) comprend la mise en sourdine (420) d'une partie des données audio comprenant le SOI comprenant les caractéristiques d'identification vocale détectées.

7. Procédé selon la revendication 1, dans lequel la classification comprend l'annotation du signal audio avec l'un quelconque des paramètres suivants : utilisation de l'outil, durée d'utilisation, fréquence, force d'utilisation, variation de la force d'utilisation.

8. Procédé selon l'une quelconque des revendications précédentes, comprenant en outre le fait de détecter si un SOI comprend des caractéristiques audio cardiaques.

9. Procédé selon la revendication précédente, comprenant la mesure d'un rythme cardiaque sur la base du signal audio détecté comprenant les caractéristiques audio cardiaques.

10. Procédé selon l'une quelconque des revendications 8 à 9, comprenant en outre la sortie du signal audio comprenant les caractéristiques audio cardiaques détectées.

11. Produit-programme d'ordinateur comprenant un programme exécutable par ordinateur d'instructions permettant d'effectuer, lorsqu'il est exécuté sur un ordinateur, les étapes du procédé selon l'une quelconque des revendications précédentes.

12. Système d'anonymisation d'un flux de données audio dans un environnement hospitalier, le système comprenant
- un moyen d'enregistrement audio (100) pour obtenir des données audio, dans lequel les données audio comprennent un ou plusieurs signaux audio (S1, S2, S3) ;
- un processeur audio configuré pour effectuer les étapes du procédé selon l'une quelconque des revendications 1 à 10.
